(19) Europäisches Patentamt
European Patent Office
Office européen des brevets

(11) EP 0 946 516 B1

(12) **FASCICULE DE BREVET EUROPEEN**

(45) Date de publication et mention de la délivrance du brevet:
**10.04.2002 Bulletin 2002/15**

(21) Numéro de dépôt: **97912295.9**

(22) Date de dépôt: **31.10.1997**

(51) Int Cl.[7]: **C07D 233/54**, A61K 7/48, C07K 5/06

(86) Numéro de dépôt international:
**PCT/FR97/01957**

(87) Numéro de publication internationale:
**WO 98/24770 (11.06.1998 Gazette 1998/23)**

(54) **NOUVEAUX DERIVES D'HISTIDINE, PROCEDE DE PREPARATION ET LEUR UTILISATION COMME AGENTS ANTI-RADICAUX LIBRES**

NEUE HISTIDINDERIVATE, VERFAHREN ZU IHRER HERSTELLUNG UND IHRE VERWENDUNG ALS MITTEL GEGEN FREIE RADIKALE

NEW HISTIDINE DERIVATIVES, PREPARATION PROCESS, AND THEIR USE AS FREE ANTIRADICAL AGENTS

(84) Etats contractants désignés:
**AT BE CH DE ES FR GB IT LI NL SE**

(30) Priorité: **04.12.1996 FR 9614880**

(43) Date de publication de la demande:
**06.10.1999 Bulletin 1999/40**

(73) Titulaire: **L'OREAL**
**75008 Paris (FR)**

(72) Inventeurs:
- **PHILIPPE, Michel**
  **F-91320 Wissous (FR)**
- **BORDIER, Thierry**
  **F-93290 Tremblay en France (FR)**

(74) Mandataire: **Dodin, Catherine et al**
**L'OREAL-DPI**
**6 rue Bertrand Sincholle**
**92585 Clichy Cédex (FR)**

(56) Documents cités:
**EP-A- 0 500 332**
**WO-A-90/06102**
**FR-A- 2 668 365**

Remarques:
Le dossier contient des informations techniques présentées postérieurement au dépôt de la demande et ne figurant pas dans le présent fascicule.

EP 0 946 516 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention a pour objet de nouveaux dérivés d'histidine et leur procédé de préparation. Elle a également pour objet des compositions cosmétiques ou dermatologiques comprenant ces composés. Elle a encore plus particulièrement pour objet l'utilisation de ces composés comme agents anti-radicaux libres.

Le rayonnement solaire, la chaleur, la pollution atmosphérique et notamment les fumées et le tabac sont connus pour entraîner la formation des radicaux libres. Ils proviennent en grande partie de l'oxygène moléculaire.
On peut citer les radicaux libres suivants:

- l'oxygène singulet, très oxydant, très toxique et d'une durée de vie très courte, produit de l'excitation de l'oxygène moléculaire par les photons de la lumière;
- le radical anion superoxyde, produit de l'addition d'un électron sur l'oxygène et pouvant donner lieu à la production des radicaux hydroxyles très réactifs;
- le radical hydroxyle, très oxydant et le plus toxique pour les cellules.

La formation de ces espèces radicalaires conduisent notamment à l'oxydation des lipides cutanés.
Les cellules vivantes, notamment celles de la peau, du cuir chevelu et de certaines muqueuses, sont particulièrement sensibles à ces radicaux libres, ce qui se traduit par un vieillissement accéléré de la peau, avec un teint manquant d'éclat et une formation précoce de rides ou de ridules, et aussi par une diminution de la vigueur et un aspect terne des cheveux.
Il est donc paru particulièrement important de protéger la peau, les cheveux et les muqueuses de ces radicaux libres.
Il est connu que certains anti-oxydants sont susceptibles d'inhiber la formation de radicaux libres.
Ainsi, la camosine, ou N-β-alanyl-L-histidine, qui est un dipeptide naturel se trouvant dans les muscles de nombreux vertébrés, est connue pour son activité anti-radicaux libres, en particulier anti oxygène singulet. (E. Decker et H. Faraji, JAOCS, vol 67, n° 10, 650-652, 1990). Son utilisation comme agent-antioxydant ou comme agent anti-radicaux libre en cosmétique est aussi connue par la demande WO-A-92/09298 Cependant, la carnosine présente au contact de la peau des problèmes de dégradation causés par les enzymes présentes dans la peau et notamment les protéases, ce qui conduit à une perte sensible de son activité.

Des dérivés de carnosine sont aussi connus, comme par exemple les dérivés N-acylés de là carnosine décrits dans le brevet FR-C-2496660. Des produits à activité antioxydante, obtenus par couplage d'acides gras et de carnosine, utilisés dans des préparations cosmétiques ont été décrits dans les demandes FR-A-2668365, WO 90/06102 ou EP-A-0500332. Toutefois, de tels dérivés de carnosine présentent aussi le même problème d'instabilité en présence des enzymes présentes dans la peau.

La demanderesse a découvert de façon inattendue de nouveaux dérivés d'histidine présentant une meilleure stabilité au contact des enzymes présentes dans la peau, et notamment des protéases, que celle des dérivés connus de l'art antérieur tout en ayant une bonne activité anti-radicaux libre et notamment ayant une bonne propriété d'efficacité dans la désactivation de l'oxygène singulet. Ils peuvent donc être utilisés en cosmétique et en pharmacie : ils sont facilement applicables sur la peau.
La demanderesse a constaté que de tels résultats pouvaient être obtenus avec des dérivés lipodipeptidiques de l'histidine à fonction carbamate.

L'invention a donc pour objet de nouveaux dérivés d'histidine répondant à la formule générale (I) suivante :

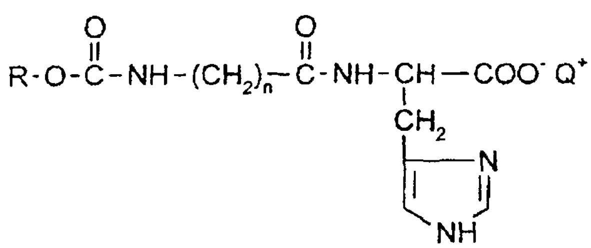

(I)

dans laquelle :

R désigne un radical alkyle, linéaire ou ramifié, saturé ou insaturé, ayant de 6 à 22 atomes de carbone,
n est un entier allant de 1 à 16,
$Q^+$ représente $H^+$ ou un cation organique ou minéral.

et les sels d'addition avec un acide.

Selon l'invention, R désigne de préférence un radical alkyle, linéaire ou ramifié, saturé, ayant de 8 à 18 atomes de carbone et n est un entier allant de 1 à 11.

Le cation organique peut être choisi parmi les ammoniums comportant un reste choisi parmi les aminoacides basiques tels que la lysine, l'arginine, ou bien encore parmi les amino-alcools tels que la glucamine, la N-méthyl glucamine, l'amino-3 propanediol-1,2.

Le cation minéral peut être choisi parmi les sels alcalins ou alcalino-terreux tels que $Na^+$, $K^+$, ou peut être l'ion $NH_4^+$.

Les sels d'addition avec un acide sont choisis parmi les chlorhydrates, les bromhydrates, les sulfates, les tartrates et les acétates.

Les composés de formule (I) comportant dans leur structure chimique un carbone asymétrique comprennent les composés à configuration D, à configuration L ou à configuration D,L.

Parmi les composés préférés correspondant à la formule générale (I), on peut notamment citer :

- la N-octyloxycarbonyl-β-alanyl-L-histidine,
- la N-dodecyloxycarbonyl-β-alanyl-L-histidine,
- le chlorhydrate de N-2-éthylhexyloxycarbonyl-β-alanyl-L-histidine,
- la N-hexadécyloxycarbonyl-β-alanyl-L-histidine.

La présente invention a également pour objet le procédé de préparation des composés de formule (I). Ce procédé consiste à faire réagir dans un solvant inerte, un composé de formule (II)

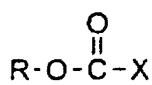

(II)

X représentant un atome d'halogène, notamment un atome de chlore, ou un radical provenant d'un imidazole tel que celui de formule (III):

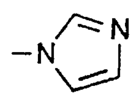

(III)

et R ayant la même signification indiquée dans la formule (I) ci-dessus,

(A) soit avec la carnosine, ou bien

(B) soit, dans une première étape, avec un amino acide de formule :

$$H_2N\text{-}(CH_2)_n\text{-}COOH$$

pour former un composé de formule (IV) suivante :

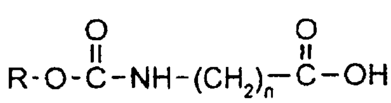

(IV)

R et n ayant les mêmes significations que celles indiquées dans la formule (I) définie ci-dessus,
et, dans une deuxième étape, à faire réagir l'histidine avec le composé de formule (IV), en présence d'un agent de couplage.

On entend par agent de couplage tout composé susceptible de substituer le groupe OH du composé de formule (IV), puis d'être substitué ensuite par l'histidine. Des agents de couplage sont cités dans "Advanced organic

chemistry, J March, 3ème édition, 1985, page 372. On peut notamment utiliser comme agent de couplage le 2-(5-norbornène-2,3-dicarboximido)-1,1,3,3 tétraméthyluronium tétrafluoroborate.

La carnosine et l'histidine de départ, comportant chacune un carbone asymétrique, sont utilisées dans la forme optique, pure ou en mélange (D; L; D,L) selon la forme optique désirée du composé de formule (I).
Comme solvant inerte, on peut utiliser le dichlorométhane, le dichloro-1,2 éthane, le trichloro-1,1,1 éthane, le chloroforme, l'acétonitrile, le toluène, le dioxane, le tétrahydrofurane, le diméthoxy-1,2 éthane, le cyclohexane, l'eau ou un mélange de ces solvants.
La réaction est effectuée à une température comprise de préférence entre -10°C et + 40°C, et plus préférentiellement entre 20°C et 30°C.
La réaction peut être effectuée en présence d'une base. Celle-ci peut être choisie parmi les hydroxydes de métaux alcalins ou alcalino-terreux, l'hydrogénocarbonate de sodium, les alcoolates de métaux alcalins, les hydrures alcalins, les amines tertiaires telles que la pyridine ou la triéthylamine. On utilise de préférence l'hydrogénocarbonate de sodium.

La présente invention a également pour objet une composition comprenant, dans un milieu physiologiquement acceptable, un composé de formule (I) tel que défini ci-dessus.
La composition comprenant ledit composé peut se présenter notamment sous forme d'une composition cosmétique ou pharmaceutique comprenant respectivement un milieu cosmétiquement ou pharmaceutiquement acceptable.
Dans les compositions selon l'invention, les composés de formule (I) sont en général présents à une concentration de 0,01% à 15% en poids et de préférence de 0,1% à 5% en poids par rapport au poids total de la composition.

Ces compositions peuvent être préparées selon les méthodes usuelles connues de l'homme de l'art. Elles peuvent être sous forme de lotion, de gel, d'émulsion eau-dans-huile ou huile-dans-eau, de microémulsion, de lait ou de crème, de poudre, de pâtes, de stick solide, de spray ou de mousse aérosol.

L'invention a également pour objet l'utilisation des composés de formule (I) comme agent anti-radicaux libres, et notamment comme agent anti-radicaux libres désactivant l'oxygène singulet, et notamment dans une composition cosmétique ou pharmaceutique.

L'invention concerne également l'utilisation des composés de formule (I) dans une composition cosmétique ou pharmaceutique pour le traitement des matières kératiniques contre les effets du vieillissement.
Par matières kératiniques, on entend la peau, les cheveux, les ongles, les poils, les muqueuses et les semi-muqueuses telles que les lèvres.

La demanderesse a découvert en outre, de manière surprenante que les composés de formule (I) constituaient des lipides amphiphiles anioniques susceptibles de former des vésicules lipidiques stables.
De façon connue, les vésicules lipidiques sont en général caractérisées par une membrane lipidique constituée par des feuillets sensiblement concentriques comportant une ou plusieurs couches multimoléculaires encapsulant une phase liquide. Cette phase liquide est couramment une phase aqueuse. Ces vésicules sont de façon connue, préparées sous forme de dispersion dans une phase aqueuse. On trouvera une liste non limitative de divers modes de préparation dans "Les liposomes en biologie cellulaire et pharmacologie" - Editions INSERM - John LIBLEY, Eurotext, 1987, pages 6 à 18.

La présente invention a donc pour objet une dispersion aqueuse de vésicules lipidiques comprenant une membrane lipidique formée à partir d'au moins un composé de formule (I) tel que défini ci-dessus.

De façon préférentielle, les vésicules lipidiques conformes à l'invention comportent une membrane lipidique encapsulant une phase aqueuse.

La présente invention a aussi pour objet une composition cosmétique ou pharmaceutique contenant une dispersion aqueuse de vésicules lipidiques comprenant une membrane lipidique formée à partir d'au moins un composé de formule (I).

Un autre objet de l'invention consiste également en l'utilisation des composés de formule (1) telle que définie ci-dessus comme lipide amphiphile susceptible de former des vésicules lipidiques.

Selon l'invention, tous les lipides amphiphiles ioniques et/ou non-ioniques susceptibles de former des vésicules stables, seuls ou en mélange avec des additifs ayant pour fonction de diminuer la perméabilité des membranes des vésicules et d'améliorer leur stabilité, peuvent être utilisés en mélange avec les composés de formule (I) pour constituer les membranes lipidiques des vésicules selon l'invention. La phase lipidique constitutive des membranes des vésicules de la dispersion selon l'invention peuvent donc comprendre, de façon connue, au moins un lipide amphiphile choisi dans le groupe formé par les lipides amphiphiles non-ioniques et les lipides amphiphiles ioniques.

Les lipides amphiphiles non-ioniques utilisés avec les composés de formule (I) pour former la membrane lipidique des vésicules selon l'invention peuvent être choisis parmi :

(1) les dérivés du glycérol de formule : $R^{10}O\text{-}[\text{-}C_3H_5\text{-}(OH)O\text{-}]_q\text{-}H$
dans laquelle :

- $-C_3H_5(OH)O-$ est représenté par les structures suivantes prises en mélange ou séparément :

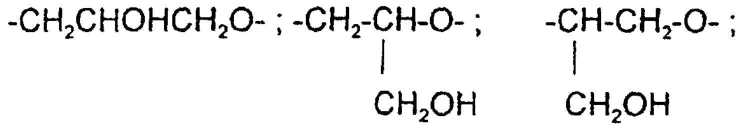

- q est une valeur statistique moyenne comprise entre 2 et 6 ;
- $R^{10}$ représente :

  (a) une chaîne aliphatique, linéaire ou ramifiée, contenant de 12 à 18 atomes de carbone ;
  (b) un reste $R^{11}CO$, où $R^{11}$ est un radical aliphatique, linéaire ou ramifié, en $C_{11}$-$C_{17}$ ;
  (c) un reste $R^{12}$-[-$OC_2H_3(R^{13})$-]-, où :

  - $R^{12}$ peut prendre la signification (a) ou (b) donnée pour $R^{10}$ ;
  - $OC_2H_3(R^{13})$- est représenté par les structures suivantes, prises en mélange ou séparément :

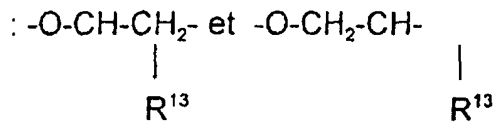

où $R^{13}$ prend la signification (a) donnée pour $R^{10}$ ;

(2) les alcools gras polyoxyéthylénés, les stérols polyoxyéthylénés ;
(3) les esters de polyols éventuellement polyoxyéthylénés ;
(4) les glycolipides naturels ou synthétiques ; et
(5) le stéarate de polyglycérol oxyéthyléné.
(6) les dérivés du glycérol décrits dans la demande de brevet PCT n° 92/08685 et répondant à la formule (V) :

$$HOCH_2\text{-}CH(OH)\text{-}CH_2\text{-}O\text{-}[\text{-}CH_2\text{-}CH(R^{14})\text{-}O\text{-}]_p\text{-}H \qquad (V)$$

dans laquelle $R^{14}$ représente un radical alkyle linéaire en $C_{14}$ à $C_{18}$ ou un groupement $-CH_2Y$ dans lequel Y est $-OR^{15}$, $R^{15}$ représentant un radical alkyle linéaire en $C_{10}$-$C_{18}$, et de préférence en $C_{16}$, et p représente une valeur statistique moyenne supérieure à 1 et au plus égale à 3, et, en outre, lorsque $R^{14}$ est-$CH_2Y$, p peut également représenter une valeur entière égale à 2;
(7) les esters et les éthers d'acide gras d'α-butylglucoside qui peuvent être soit des mélanges d'esters et/ou des mélanges d'éthers de différents acides gras d'α-butylglucoside dont les différentes chaînes grasses comportent, l'une par rapport à l'autre, un nombre d'atomes de carbone voisin (par exemple différent de 1 ou 2) soit des mélanges de mono-, di- tri- ou polyesters et/ou des mélanges de mono-, di- tri- polyéthers d'un même acide gras d'α-butylglucoside.

Les esters et les éthers d'acide gras d'α-butylglucoside utilisés selon l'invention comportent de préférence une chaîne grasse ayant de 8 à 24 atomes de carbone, plus préférentiellement de 12 à 22 atomes de carbone et plus particulièrement de 14 à 18 atomes de carbone.
On peut citer par exemple, les esters et les éthers d'acide laurique ($C_{12}$), myristique ($C_{14}$), palmitique ($C_{16}$), stéarique ($C_{18}$), béhénique ($C_{22}$) d'α-butylglucoside.
On utilise plus particulièrement un mélange de mono- et de diester d'acide palmitique d'α-butylglucoside.
Les esters et les éthers d'acide gras d'α-butylglucoside conformes à l'invention peuvent être préparés à partir d'α-butylglucoside obtenu selon le procédé de fabrication enzymatique décrit dans la demande de brevet FR-A-2680373 qui consiste à mettre en contact le butanol avec de l'amidon, des maltodextrines ou du maltose en présence d'une préparation enzymatique purifiée présentant une activité d'α-transglucosylation. Les esters et les éthers d'acide gras d'α-butylglucoside peuvent être synthétisés en faisant réagir l'acide gras ou le mélange d'acide gras correspondants avec l'α-butylglucoside selon des procédés classiques.
Les lipides amphiphiles ioniques utilisés en association avec les composés de formule (I) pour former la membrane des vésicules selon l'invention peuvent être choisis parmi :

(1) les lipides amphiphiles anioniques suivants :

- les phospholipides naturels tels que la lécithine d'oeuf ou de soja, la sphingomyéline, la phosphatidylsérine, la dipalmitoylphosphatidylcholine et les lecithines hydrogénées., les phospholipides modifiés par voie chimique ou enzymatique, et les phospholipides de synthèse;
- les composés anioniques de formule (VI) :

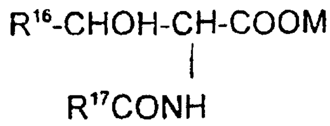

(VI)

dans laquelle :

$R^{16}$ représente un radical alkyle ou alcényle en $C_7$-$C_{21}$,
$R^{17}$ représente un radical hydrocarboné, saturé ou insaturé, en $C_7$-$C_{31}$,
M représente H, Na, K, NH4 ou un ion ammonium substitué dérivé d'une amine;

- les composés anioniques tels que les esters phosphoriques d'alcool gras, notamment le dicétylphosphate et le dimyristylphosphate sous forme d'acide ou de sels alcalins, l'acide heptylnonylbenzène sulfonique, le sulfate de cholestérol acide ou le phosphate de cholestérol acide, ainsi que leur sels alcalins, les lysolécitines, les alkylsulfates tels que le cétylsulfate de sodium, les gangliosides, les acylglutamates mono et disodique, et en particulier les sels mono- et disodiques de l'acide N-stéaroylglutamique, les sels de sodium de l'acide phosphatidique, les phosphoaminolipides, les phospholipides naturels,

(2) les lipides amphiphiles cationiques suivants :

- les composés cationiques ayant la formule (VII) :

$$(R^{18})(R^{19})N^+(R^{20})(R^{21})\ X^- \qquad (VII)$$

dans laquelle $R^{18}$ et $R^{19}$, identiques ou différents, représentent des radicaux alkyles en $C_{12}$-$C_{20}$ et $R^{20}$ et $R^{21}$, identiques ou différents, des radicaux alkyles en $C_1$-$C_4$,
- les amines à longue chaîne et leurs dérivés ammonium quaternaire, les esters d'aminoalcools à longue chaîne et leurs sels et dérivés ammonium quaternaire,
- les lipides polymérisables, comme décrits par RIINGSDORF et autres, dans "Angewandte Chemie", vol 27, n° 1, Janvier 1988, pages 129-137.

On peut, de façon connue, incorporer dans la phase lipidique constituant la membrane lipidique des vésicules de l'invention, au moins un additif qui a pour fonction principale de diminuer la perméabilité des vésicules, de prévenir leur floculation et leur fusion et d'augmenter le taux d'encapsulation.
Selon un mode préférentiel de l'invention, on peut ajouter à la phase lipidique au moins un additif choisi, de préférence, dans le groupe formé par :

- les stérols et notamment les phytostérols et le cholestérol,
- les alcools et diols à longue chaîne,
- les amines à longue chaîne et leurs dérivés ammonium quaternaire.

Ces additifs peuvent éventuellement avoir une activité cosmétique et/ou dermopharmaceutique. C'est, par exemple, le cas du cholestérol.
La phase continue aqueuse de la dispersion de vésicules selon l'invention peut être constituée par de l'eau, ou un mélange d'eau et d'au moins un solvant miscible à l'eau tels que les alcools en $C_1$-$C_7$ et les polyols d'alkyle en $C_2$-$C_5$. Cette phase aqueuse peut également contenir des composés en solution, tels que des sucres, des sels organiques ou minéraux ou des polymères. Elle peut également contenir une dispersion de gouttelettes d'un liquide non

miscible à l'eau que les vésicules stabilisent, de sorte qu'il n'est pas nécessaire d'introduire pour cette stabilisation un émulsionnant. Ce liquide non miscible peut être choisi dans le groupe formé par les huiles animales ou végétales, les huiles essentielles naturelles ou synthétiques, les hydrocarbures, les carbures halogénés, les silicones, les esters d'acide minéral et d'un alcool, les éthers et les polyéthers. Des exemples de liquides non miscibles à l'eau sont mentionnés dans le brevet européen EP-A-455 528.

Les compositions contenant les composés selon l'invention peuvent également comporter, de façon connue, un ou plusieurs composés actifs ayant une activité cosmétique et/ou pharmaceutique qui, selon leurs caractéristiques de solubilité, peuvent avoir différentes localisations. Par exemple, dans le cas des dispersions de vésicules contenant une phase aqueuse encapsulée, si les actifs sont liposoiubles, ils peuvent être présents dans la phase lipidique constituant le(s) feuillet(s) des vésicules ou dans les gouttelettes de liquide non miscible à l'eau stabilisées par les vésicules. Si les actifs sont hydrosolubles, ils peuvent être présents dans la phase aqueuse encapsulée des vésicules ou dans la phase aqueuse continue de la dispersion. Si les actifs sont amphiphiles, ils se répartissent entre la phase lipidique et la phase aqueuse encapsulée avec un coefficient de partage, qui varie selon la nature de l'actif amphiphile et les compositions respectives de la phase lipidique et de la phase aqueuse encapsulée. De façon générale, les actifs sont mis en place dans la phase lipidique des feuillets et/ou dans la phase encapsulée par les feuillets.

Les compositions selon l'invention peuvent également comprendre, de façon connue, des additifs de formulation n'ayant ni activité cosmétique, ni activité pharmaceutique propre, mais utiles pour la formulation des compositions. Parmi ces additifs, on peut citer par exemple les gélifiants, les polymères, les conservateurs, les colorants, les opacifiants et les parfums.

Les compositions cosmétiques ou pharmaceutiques selon l'invention peuvent se présenter notamment sous forme de shampooings ou d'après shampooing, de compositions nettoyantes, de crèmes pour le soin de la peau ou des cheveux, de compositions anti-solaires, de crèmes ou de mousse après-rasage, de déodorants corporels, de composition à usage bucco-dentaire, de compositions tinctoriales capillaires, ou encore de composition de maquillage par exemple.

Les exemples donnés ci-après, à titre illustratif et nullement limitatif, permettront de mieux comprendre l'invention.

**Exemple 1: Préparation de la N-octyloxycarbonyl-β-alanyl-L-histidine**

a) Préparation de la N-octyloxycarbonyl-β-alanine :

On a dissous 20 g (224,46 mmoles) de β-alanine dans 225 ml d'une solution de soude 1N. Puis 44 ml de chloroformiate d'octyle ont été ajoutés goutte-à-goutte, tout en maintenant le pH du milieu réactionnel supérieur à 9 par l'addition simultanée d'un équivalent molaire de soude 1 N. Après 6 heures sous agitation à température ambiante, le mélange a été acidifié par environ 1,1 équivalent molaire d'une solution d'acide chlorhydrique 3 N. Le mélange hétérogène a été extrait par 300 ml d'acétate d'éthyle. La phase organique a été lavée par 3 fois 80 ml d'eau, séchée puis évaporée à sec. Le résidu solide a été repris par de l'heptane, puis filtré et séché. On a obtenu 51 g d'un produit blanc (rendement de 93 %).
Point de fusion : 70-72 °C

| Analyse élémentaire : $C_{12}H_{23}NO_4$, PM = 245,3 | | | | |
|---|---|---|---|---|
| | **C** | **H** | **N** | **O** |
| **Calculé** | 58,75 | 9,45 | 5,71 | 26,09 |
| **Trouvé** | 58,57 | 9,44 | 5,77 | 26,25 |

Spectre RMN 200 MHz ($^1H$, DMSO d6), δ ppm:
12,030 (1H, s, COOH 12), 6,920 (1H, te, NH 9), 3,750 (2H, t, $CH_2$ 11), 3,010 (2H, m, $CH_2$ 10), 2,210 (2H, t, $CH_2$ 8), 1,090 à 1,360 (12H, m, $CH_2$ 2 à 7), 0,710 (3H, t, $CH_3$ 1).

b) Préparation de la N-octyloxycarbonyl-β-alanyl-L-histidine :

30g de N-octyloxycarbonyl-β-alanine ont été dissous dans 300 ml de diméthylformamide et 1 équivalent molaire de triéthylamine. Puis 1 équivalent molaire de 2-(5-norbornène-2,3-dicarboximido)-1,1,3,3 tétraméthyluronium tétrafluoroborate a été ajouté. Le mélange a été ensuite agité pendant 1 heure à la température de 25 °C. Puis on a ajouté goutte-à-goutte une solution contenant 1,2 équivalent molaire d'histidine et 1,2 équivalent molaire de soude dans 100 ml d'eau, en maintenant la température inférieure à 30 °C. Après 6 heures d'agitation, le mélange a été

neutralisé par 1 équivalent molaire d'acide chlorhydrique 5 N suivi de 100 ml d'eau. Après 30 minutes d'agitation, le mélange a été versé dans 2 litres d'acétone. Le précipité blanc formé a été filtré et séché. Le produit brut a été dissous dans 10ml/g d'éthanol à 96 % au reflux. La solution a été filtrée à chaud puis le produit a cristallisé à température ambiante. Après traitement du précipité, on a obtenu 29,4 g d'un produit cristallin blanc (rendement 63 %).
Point de fusion : 188-190 °C

| Analyse élémentaire : $C_{18} H_{30} N_4 O_5$, 2/3 $H_2O$ ; PM = 394,4 | | | | |
|---|---|---|---|---|
| | **C** | **H** | **N** | **O** |
| **Calculé** | 54,74 | 8,19 | 14,24 | 22,98 |
| **Trouvé** | 54,75 | 7,94 | 14,19 | 22,96 |

Spectre RMN 400 MHz (1H, DMSO d6), δ (ppm) :
8,100 (1H,d, NH entre 12 et 13), 7,580 (1H, s, CH 17), 6,970 (1H, t, NH entre 9 et 10), 6,810 (1H, s, CH 16), 4,400 (1H, m, CH 13), 3,910 (2H, t, CH2 8), 3,150 (2H, m, CH2 10), 2,950 (1H, dd, CH2 14), 2,840 (1H, dd, CH2 14), 2,270 (2H, t, CH2 11), 1,510 (2H, m, CH2 7), 1,250 (10H, m, CH2 2 à 6), 0,860 (3H, t, CH3 1).

**Exemple 2 : Préparation de la N-dodécyloxycarbonyl-β-alanyl-L-histidine**

a) Préparation de N-dodécyloxycarbonyl-β-alanine :

Le composé a été préparé selon le même mode opératoire de l'exemple 1 a) en utilisant 55,6 g de chloroformiate de dodécyle. On a obtenu 27 g d'un produit blanc (rendement de 40 %).
Point de fusion : 88-90 °C

| Analyse élémentaire : $C_{16} H_{31} N O_4$; PM = 301,43 | | | | |
|---|---|---|---|---|
| | **C** | **H** | **N** | **O** |
| **Calculé** | 63,69 | 10,28 | 4,64 | 21,23 |
| **Trouvé** | 63,68 | 10,33 | 4,60 | 21,31 |

Spectre RMN 200 MHz ($^{13}C$, $CDCl_3$), δ ppm:
177,42 C-16, 156,86 C-13, 65,34 C-12, 36,28 C-14, 34,33 C-15, 31,88 C-11, de 28,92 à 29,61 C-4 à C-10, 25,81 C-3, 22,66 C-2, 14,09 C-1.

b) Préparation de la N-dodécyloxycarbonyl-β-alanyl-L-histidine:

Le composé a été préparé selon le même mode opératoire de l'exemple 1 b), en utilisant 30 g de N-dodécyloxycarbonyl-β-alanine. On a obtenu 29,7 g d'un produit cristallin blanc (rendement de 68 %).
Point de fusion : 168-172 °C.

| Analyse élémentaire : $C_{22} H_{38} N_4 O_5$ ; PM = 438,572 | | | | |
|---|---|---|---|---|
| | **C** | **H** | **N** | **O** |
| **Calculé** | 60,25 | 8,73 | 12,77 | 18,24 |
| **Trouvé** | 59,98 | 8,92 | 12,62 | 17,93 |

Spectre RMN 400 MHz (1H, DMSO d6), δ (ppm):
8,120 (1H, d, NH 18), 7,590 (1H, s, CH 24), 7,010 (1H, t, NH 14), 6,810 (1H, s, CH 22), 4,400 (1H, m, CH 19), 3,900 (2H, t, CH2 12), 3,140 (2H, m, CH2 15), 2,940 (1H, dd, CH2 20), 2,830 (1H, dd, CH2 20), 2,260 (2H, t, CH2 16), 1,510 (2H, m, CH2 11), 1,260 (18H, m, CH2 2 à 10), 0,850 (3H, t, CH3 1).

**Exemple 3 : Préparation du chlorhydrate de N-2-éthylhexyloxycarbonyl-β-alanyl-L-histidine**

2 g de L-carnosine ont été dissous dans 10 ml d'eau et 10 ml de tétrahydrofurane en présence de 3,5 ml d'une solution de soude à 10 %. 1,7 g de chloroformiate de 2-éthylhexyle a été ajouté goutte-à-goutte au mélange

préalablement refroidi à 10 °C. Une solution de soude a été simultanément introduite pour maintenir le pH du mélange supérieur à 9. Après 6 heures à la température ambiante, le mélange a été acidifié par de l'acide chlorhydrique N/3 à pH égal à 2, puis précipité dans l'acétone. Après filtration et séchage, on a obtenu 1,2 g d'un produit blanc (rendement de 40 %).

| Analyse élémentaire : $C_{18}H_{30}N_4O_5$, HCl ; PM = 418,7 | | | | | |
|---|---|---|---|---|---|
| | **C** | **H** | **N** | **O** | **Cl** |
| **Calculé** | 51,55 | 7,40 | 13,36 | 19,09 | 8,47 |
| **Trouvé** | 51,81 | 7,61 | 13,48 | 19,67 | 8,37 |

Spectre RMN 400 MHz ($^1H$, DMSO d6 + AcOD), δ (ppm):
8,830 (1H, d, H 17), 7,280 (1H, s, H 16), 4,550 (1H, m, CH 13), 3,810 (2H, m, CH2 8), 2,960 à 3,170 (4H, m, CH2 10 et 14), 2,280 (2H, t, CH2 11), 1,430 (1H, m, CH 7), 1,190 à 1,290 (8H, m, CH2 2,3,4,5), 0,760 à 0,810 (6H, 2t, CH3 1 et 6).

**Exemple 4 : Exemple comparatif**

On a comparé l'activité anti-oxygène singulet du composé de l'exemple 1 à celle du N-octanoyl-β-alanyl-L-histidine (appelé composé A) de l'état de la technique, selon le principe suivant, aussi décrit dans Pure & Appl. Chem., vol 62, n° 8, 1467-1476 (1990).
L'oxygène singulet dans toutes les expériences a été généré par une irradiation à 437 nm du $Ru(bipy)_3$ Cl (chlorure de tris-(2,2'-bipyridyl) ruthénium, hexahydrate) en solution dans le méthanol deutérié.
On a déterminé la somme des constantes d'inhibition physique ($k_q$) et de réaction chimique ($k_r$) de l'oxygène singulet avec les composés testés.
Les résultats de désactivation de l'oxygène singulet peuvent être analysés par mesure de luminescence (émission infra-rouge) de l'oxygène singulet à 1270 nm, et par le suivi des cinétiques de disparition de l'oxygène. L'exploitation des résultats est basée sur le principe de l'analyse de type Stern-Volmer : le signal obtenu est mesuré en l'absence (So) et en (S) présence du composé testé, à différentes concentrations dans la solution. Le rapport So/S permet de déterminer la valeur de ($k_r + k_q$) selon la relation suivante :

$$So/S = \phi_o/\phi = (k_r + k_q) \times \tau_o \times [P]$$

relation dans laquelle :

[P] désigne la concentration en composé testé dans la solution,
$\phi_o$ désigne le rendement quantique de l'émission en l'absence de composé,
$\phi$ désigne le rendement quantique de l'émission en présence de composé,
$\tau_o$ désigne la durée de vie de l'oxygène singulet dans le méthanol deutérié (270 μs),
$k_r + k_q$ désigne la somme des constantes de vitesse de la réaction chimique et de l'inhibition physique de l'oxygène singulet.

On a obtenu les résultats suivants :

| **Composé** | **($k_r + k_q$) en $l.mol^{-1}.s^{-1}$** |
|---|---|
| exemple 1 (invention) | $8,8 \times 10^6 \pm 0,3$ |
| composé A (hors invention) | $7,4 \times 10^6 \pm 0,4$ |

Les résultats obtenus montrent que pour le composé selon l'invention la somme des constantes ($k_r + k_q$) est supérieure à celle du composé A de l'état de la technique : le composé selon l'invention (exemple 1) a donc une activité anti-oxygène singulet significativement supérieure à celle du composé de l'état de la technique (composé A). Le composé de l'exemple 1 possède une activité anti-radicaux libres supérieure à celle du composé A.
On décrit ci-après le mode de préparation du composé A, le N-octanoyl-β-alanyl-L-histidine :
On a dissous 2 g (8,84 mmoles) de L-carnosine dans 10 ml d'eau et 10 ml de tétrahydrofurane en présence de 3,5 ml d'une solution de soude à 10%. Puis on a ajouté goutte-à-goutte 1,5 ml de chlorure de capryloyle au mélange précédent refroidi à 10 °C. Après une nuit à la température ambiante, le mélange réactionnel a été précipité dans de l'acétone.

Le produit brut obtenu a été recristallisé dans de l'éthanol. Après filtration, lavage et séchage, on a obtenu 1,8 g d'un produit blanc (rendement de 57 %).

| Analyse élémentaire : $C_{17} H_{28} N_4 O_4$ ; PM = 352,437 | | | | |
|---|---|---|---|---|
| | **C** | **H** | **N** | **O** |
| **Calculé** | 57,94 | 8,01 | 15,9 | 18,16 |
| **Trouvé** | 57,40 | 7,92 | 15,19 | 18,48 |

Spectre RMN 500 MHz ($^1H$, DMSO d6), δ (ppm) :
8,060 (1H, d, NH 13), 7,700 (1H, t, NH 9), 7,570 (1H, s, H 17), 6,810 (1H, s, H 18), 4,390 (1H, m, CH 14), 3,200 (2H, m, CH2 10), 2,810 à 2,970 (2H, m, CH2 15), 2,260 (2H, t, CH2 11), 2,020 (2H, t, CH2 7), 1,470 (2H, m, CH2 6), 1,230 à 1,280 (8H, m, CH2 2 à 5), 0,860 (3H, t, CH3 1).

**Exemple 5**

On a préparé une émulsion huile-dans-eau ayant la composition suivante :

- composé de l'exemple 2 0,5 g
- néopentanoate d'isostéaryle 10 g
- triglycérides d'acide caprylique et d'acide caprique 8 g
- huile de paraffine 5 g
- stéarate de poléthylène glycol polyglycérolé à 4 moles d'éthylène glycol et 2 moles de glycérol sous la dénomination "Hostacerin DGS" par la société HOECHST 2 g
- mélange d'ester de l'acide phosphorique et de l'éther d'alcool oléique et de polyéthylèneglycol vendu sous la dénomination "Crofados N.10 acid" par la société CRODA 4 g
- glycérine 4 g
- stéarate de glycéryle 1,3 g
- agent épaississant 0,25 g
- eau qsp 100 g

On a ainsi obtenu une crème qui convient parfaitement pour le soin quotidien du visage.

**Exemple 6**

On a préparé une émulsion huile-dans-eau ayant la composition suivante :

- composé de l'exemple 1 g
- palmitate d'octyle 12 g
- stéarate de polyéthylène glycol à 100 mole d'éthylène glycol 1,3 g
- sorbitane de glycéryle 0,4 g
- acide stéarique 1 g
- agent épaississant 0,4 g
- eau qsp 100 g

On a ainsi obtenu une crème pour peau normale à tendance grasse qui s'applique facilement sur le visage.

**Exemple 7**

On a préparé une émulsion huile-dans-eau ayant la composition suivante :

- composé de l'exemple 2 1 g
- octanoate d'octyle 10 g
- citrate de dicapryle 8 g
- stéarate de glycéryle 0,7 g
- éther de polyéthylèneglycol (2 moles d'éthylène glycol) et d'alcool stéarylique (Brij 72 de ICI) 0,4 g
- éther de polyéthylèneglycol (21 moles d'éthylène glycol) et d'alcool stéarylique (Brij 721 de ICI) 0,8 g

- stéarate de polyéthylèneglycol à 40 moles d'éthylèneglycol (Myrj 52 de ICI) 1,5 g
- hydrolysât de protéine de blé 0,3 g
- agent épaississant 0,28 g
- glycérine 5 g
- eau qsp 100 g

On a ainsi obtenu une crème qui peut être utilisée pour le soin quotidien du visage.

**Exemple 8**

On a préparé une émulsion huile-dans-eau ayant la composition suivante :

- composé de l'exemple 1 0,5 g
- cyclométhicone 2 g
- isostéarate d'isostéaryle 4 g
- diméthicone copolyol 2 g
- acide stéarique 0,5 g
- eau qsp 100 g

On a ainsi obtenu un fluide blanc qui s'applique facilement sur le visage.

**Exemple 9**

On a préparé une composition sous forme de dispersion vésiculaire suivante :

Phase A :

- composé de l'exemple 2 1,2 %
- cholestérol 1,8 %
- vitamine E 0,3 %

Phase B :

- eau déminéralisée 30 %
- glycérine 5 %
- lysine 0,4 %

Phase C :

- eau distillée 15 %
- conservateurs 0,3 %
- hyaluronate de sodium 0,4 %

Phase D :

- mélange d'acides polycarboxyliques (Carbopol 980 de la société Goodrich) 0,20 %
- triéthanolamine qs pH 6,5
- eau déminéralisée qsp 100 %

Les composants de la phase A ont été mélangés à 90-110 °C jusqu'à obtention d'un mélange homogène. La

phase A a été introduite sous agitation vive dans la phase B chauffée également à 90 °C. On a maintenu l'agitation et la température pendant 1 heure. Puis on a effectué 3 passages en homogénéisation haute pression à 600 bars ($6x10^7$ Pa) de la suspension obtenue. On a obtenu alors des vésicules ayant une granulométrie comprise entre 100 et 250 nm. On a introduit ensuite à température ambiante la phase C puis on y a dispersé la phase D à l'aide d'une défloculeuse.
On obtient ainsi une composition lisse qui peut être utilisée comme sérum de soin anti-âge.

**Exemple 10**

On a préparé une composition sous forme de dispersion vésiculaire suivante :

Phase A

- composé de l'exemple 2 2%
- cholestérol 3 %
- vitamine E 0,5 %

Phase B

- eau déminéralisée 35 %
- glycérine 5 %
- lysine 0,65 %

Phase C

- eau distillée 5 %
- conservateurs 0,3 %

Phase D

- huile de silicone volatile 10 %
- huile d'amande d'abricot 10 %
- conservateurs 0,1 %

Phase E

- mélange d'acides polycarboxyliques (Carbopol 980 de la société Goodrich) 0,20 %
- triéthanolamine qs pH 6,5
- eau déminéralisée qsp 100 %

La composition a été préparée selon le mode opératoire utilisé dans l'exemple 9.
La phase D a été introduite sous vive agitation dans la dispersion vésiculaire obtenue après l'incorporation de la phase C. L'ensemble a ensuite été homogénéisé à 600 bars ($6x10^7$ Pa) deux fois. La granulométrie des globules huileux était de l'ordre de 300 nm. Puis on a dispersé la phase E.
On obtient une composition qui peut être utilisée comme crème de jour pour le visage.

**Exemple 11**

On a préparé une composition sous forme de dispersion vésiculaire suivante :

Phase A

- composé de l'exemple 2 1,5 %
- cholestérol 2,7 %
- vitamine E 0,8 %
- palmitate de sorbitane 3,8 %

Phase B

- eau déminéralisée 50 %
- glycérine 5 %
- lysine 0,5 %

Phase C

- eau distillée 5 %
- conservateurs 0,3 %

Phase D

- huile de macadamia 5 %
- conservateurs 0,3 %

Phase E

- mélange d'acides polycarboxyliques (Carbopol 980 de la société Goodrich) 0,42 %
- triéthanolamine qs pH 6,5
- eau déminéralisée qsp 100 %

La composition a été préparée selon le mode opératoire utilisé dans l'exemple 10. On obtient ainsi une composition qui peut être utilisée comme crème de jour pour le visage.

## Revendications

1. Dérivé d'histidine de formule générale (I) suivante:

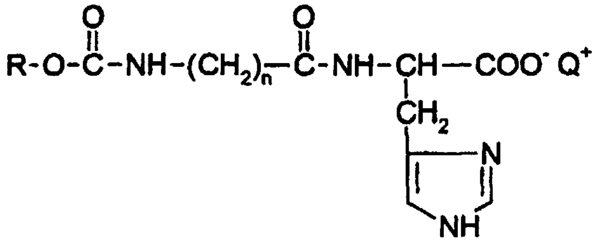

(I)

dans laquelle:

R désigne un radical alkyle, linéaire ou ramifié, saturé ou insaturé, ayant de 6 à 22 atomes de carbone,
n est un entier allant de 1 à 16,
$Q^+$ représente $H^+$ ou un cation organique ou minéral,

et les sels d'addition avec un acide,
ledit cation organique étant choisi parmi les ammoniums comportant un reste choisi parmi les aminoacides basiaues ou les amino-alcools,
ledit cation minéral étant choisi parmi le groupe formé par les sels alcalins, les sels alcalino-terreux, l'ion $NH_4^+$,
lesdits sels d'addition avec un acide étant choisis parmi les chlorhydrates, les bromhydrates, les sulfates, les tartrates et les acétates.

**2.** Dérivé d'histidine selon la revendication 1, dans lequel R désigne un radical alkyle, linéaire ou ramifié, saturé, ayant de 8 à 18 atomes de carbone.

**3.** Dérivé d'histidine selon l'une des revendications précédentes, dans lequel n est un entier allant de 1 à 11.

**4.** Dérivé d'histidinè selon l'une quelconque des revendications précédentes, choisi parmi :

- la N-octyloxycarbonyl-β-alanyl-L-histidine,
- la N-dodecyloxycarbonyl-β-alanyl-L-histidine,
- le chlorhydrate de N-2-éthylhexyloxycarbonyl-β-alanyl-L-histidine,
- la N-hexadécyloxycarbonyl-β-alanyl-L-histidine.

**5.** Procédé de préparation des composés de formule générale (I) suivante :

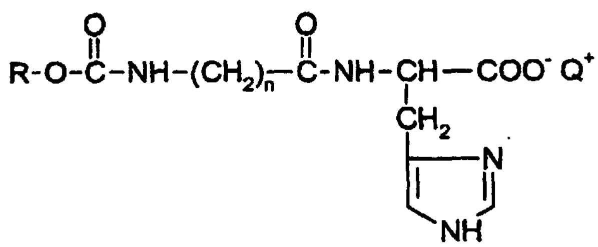 (I)

dans laquelle :

R désigne un radical alkyle, linéaire ou ramifié, saturé ou insaturé, ayant de 6 à 22 atomes de carbone,
n est un entier allant de 1 à 16,
$Q^+$ représente $H^+$ ou un cation organique ou minéral,

**caractérisé par le fait qu'**il consiste à faire réagir, dans un solvant inerte, un composé de formule (II)

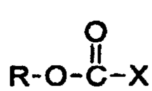 (II)

X représentant un atome d'halogène ou un radical provenant d'un imidazole,

(A) soit avec la carnosine, ou bien

(B) soit, dans une première étape, avec un amino acide de formule

$$H_2N-(CH_2)_n-COOH$$

pour former un composé de formule (IV) suivante :

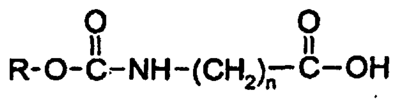

(IV)

et, dans une deuxième étape, à faire réagir l'histidine avec le composé de formule (IV), en présence d'un agent de couplage.

6. Procédé selon la revendication 5, **caractérisé par le fait que** X est un atome de chlore.

7. Composition **caractérisée par le fait qu'**elle comprend, dans un milieu physiologiquement acceptable, au moins un composé de formule (I) tel que défini dans l'une des revendications 1 à 4.

8. Composition selon la revendication 7 **caractérisée par le fait qu'**elle se présente sous la forme d'une composition cosmétique ou pharmaceutique.

9. Composition selon l'une des revendications 7 ou 8, **caractérisée par le fait qu'**elle se présente sous la forme de lotion, de gel, d'émulsion, de microémulsion, de lait ou de crème, de poudre, de pâte, de stick solide, de spray ou de mousse aérosol.

10. Composition selon l'une des revendications 7 à 9, **caractérisée par le fait que** le composé de formule (I) est présent à une concentration de 0,01% à 15% en poids par rapport au poids total de la composition.

11. Utilisation des composés de formule (I) selon l'une des revendications 1 à 4 comme agent anti-radicaux libres.

12. Utilisation des composés de formule (I) selon l'une des revendications 1 à 4 comme agent anti-radicaux libres désactivant l'oxygène singulet.

13. Utilisation des composés de formule (I) selon l'une des revendications 1 à 4 pour la préparation d'une composition cosmétique ou pharmaceutique destinée au traitement des matières kératiniques contre les effets du vieillissement.

14. Dispersion aqueuse de vésicules lipidiques, **caractérisée par le fait que** les vésicules comportent une membrane lipidique formée à partir d'au moins un composé de formule (I) selon l'une des revendications 1 à 4.

15. Dispersion selon la revendication 14, **caractérisée par le fait que** la membrane lipidique encapsule une phase aqueuse.

16. Dispersion selon l'une des revendications 14 ou 15, **caractérisée par le fait que** la phase lipidique constituant la membrane lipidique comprend en plus au moins un lipide amphiphile non ionique et/ou un lipide amphiphile ionique.

17. Dispersion selon l'une des revendications 14 à 16, **caractérisée par le fait que** la phase lipidique constituant la membrane lipidique comprend au moins un additif qui a pour fonction principale de diminuer la perméabilité des vésicules, de prévenir leur floculation et leur fusion et d'augmenter le taux d'encapsulation.

18. Dispersion selon la revendication 17, **caractérisée par le fait que** l'additif est choisi dans le groupe formé par les stérols, les alcools et diols à longue chaîne, les amines à longue chaîne et leurs dérivés ammonium quaternaire.

19. Dispersion aqueuse de vésicules selon la revendication 18, **caractérisée par le fait que** l'additif est le cholestérol.

20. Composition cosmétique ou pharmaceutique **caractérisée par le fait qu'**elle comprend, dans un milieu cosmétiquement ou pharmaceutiquement acceptable, au moins une dispersion aqueuse de vésicules selon l'une des revendications 14 à 19.

21. Utilisation d'un composé de formule (1) selon l'une des revendications 1 à 4 comme lipide amphiphile pour former

des vésicules lipidiques.

**Patentansprüche**

1. Histidinderivat der folgenden allgemeinen Formel (I):

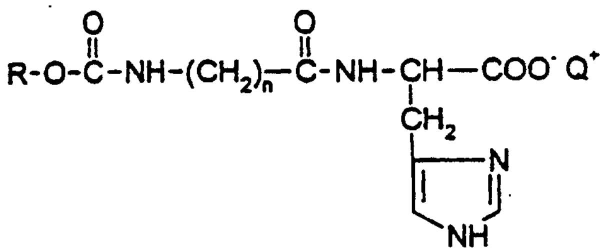

(I),

worin bedeuten:

- R eine geradkettige oder verzweigte, gesättigte oder ungesättigte Alkylgruppe mit 6 bis 22 Kohlenstoffatomen,
- n eine ganze Zahl im Bereich von 1 bis 16 und
- $Q^+$ $H^+$ oder ein organisches oder anorganisches Kation,

und die Additionssalze dieser Verbindungen mit einer Säure, wobei:

- das organische Kation unter Ammoniumverbindungen ausgewählt ist, die eine Gruppe aufweisen, die unter basischen Aminosäuren oder Aminoalkoholen ausgewählt ist,
- das anorganische Kation unter Alkalisalzen, Erdalkalisalzen und dem $NH_4^+$-Ion ausgewählt ist und
- die Additionssalze mit einer Säure unter Hydrochloriden, Hydrobromiden, Sulfaten, Tartraten und Acetaten ausgewählt sind.

2. Histidinderivat nach Anspruch 1, worin R eine geradkettige oder verzweigte, gesättigte Alkylgruppe mit 8 bis 18 Kohlenstoffatomen bedeutet.

3. Histidinderivat nach einem der vorhergehenden Ansprüche, worin n eine ganze Zahl im Bereich von 1 bis 11 bedeutet.

4. Histidinderivat nach einem der vorhergehenden Ansprüche, das ausgewählt ist unter:

- N-Octyloxycarbonyl-β-alanyl-L-histidin,
- N-Dodecyloxycarbonyl-β-alanyl-L-histidin,
- N-2-Ethylhexyloxycarbonyl-β-alanyl-L-histidin-Hydrochlorid und
- N-Hexadecyloxycarbonyl-β-alanyl-L-histidin.

5. Verfahren zur Herstellung der Verbindungen der folgenden allgemeinen Formel (I):

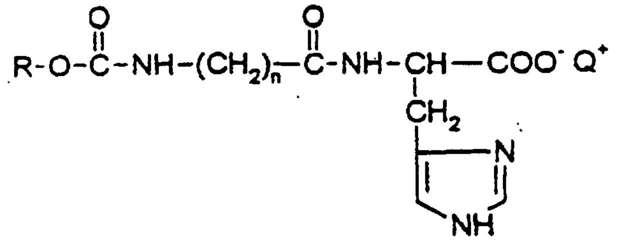

(I),

worin bedeuten:

- R eine geradkettige oder verzweigte, gesättigte oder ungesättigte Alkylgruppe mit 6 bis 22 Kohlenstoffatomen,
- n eine ganze Zahl im Bereich von 1 bis 16 und
- $Q^+$ $H^+$ oder ein organisches oder anorganisches Kation,

**dadurch gekennzeichnet, dass** es darin besteht, eine Verbindung der Formel (II)

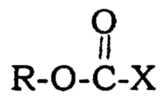

(II),

worin X ein Halogenatom oder eine Gruppe bedeutet, die von einem Imidazol stammt, in einem inerten Lösemittel entweder

(A) mit Carnosin oder
(B) in einem ersten Schritt mit einer Aminosäure der Formel

$$H_2N\text{-}(CH_2)_n\text{-}COOH$$

umzusetzen,

um eine Verbindung der folgenden Formel (IV) zu erhalten:

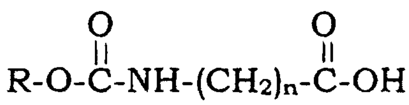

(IV),

und dann in einem zweiten Schritt Histidin mit der Verbindung der Formel (IV) in Gegenwart eines Kupplungsmittels umzusetzen.

**6.** Verfahren nach Anspruch 5, **dadurch gekennzeichnet, dass** X ein Chloratom bedeutet.

**7.** Zusammensetzung, **dadurch gekennzeichnet, dass** sie in einem physiologisch akzeptablen Medium mindestens eine wie in einem der Ansprüche 1 bis 4 definierte Verbindung der Formel (I) enthält.

**8.** Zusammensetzung nach Anspruch 7, **dadurch gekennzeichnet, dass** sie in Form einer kosmetischen oder pharmazeutischen Zusammensetzung vorliegt.

**9.** Zusammensetzung nach einem der Ansprüche 7 oder 8, **dadurch gekennzeichnet, dass** sie in Form einer Lotion, eines Gels, einer Emulsion, einer Mikroemulsion, einer Milch oder einer Creme, eines Pulvers, einer Paste, eines festen Sticks, eines Sprays oder eines Aerosolschaums vorliegt.

**10.** Zusammensetzung nach einem der Ansprüche 7 bis 9, **dadurch gekennzeichnet, dass** die Verbindung der Formel (I) in einer Konzentration im Bereich von 0,01 bis 15 Gew.-%, bezogen auf das Gesamtgewicht der Zusammensetzung, vorliegt.

**11.** Verwendung der Verbindungen der Formel (I) nach einem der Ansprüche 1 bis 4 als Radikalfänger gegen freie Radikale.

**12.** Verwendung der Verbindungen der Formel (I) nach einem der Ansprüche 1 bis 4 als Radikalfänger gegen freie Radikale, der Singulett-Sauerstoff desaktiviert.

**13.** Verwendung der Verbindungen der Formel (I) nach einem der Ansprüche 1 bis 4 zur Herstellung einer kosmetischen oder pharmazeutischen Zusammensetzung, die zur Behandlung keratinischer Materialien gegen die Wirkungen der Alterung bestimmt ist.

**14.** Wässerige Dispersion von Lipidvesikeln, **dadurch gekennzeichnet, dass** die Vesikel eine Lipidmembran aufweisen, die von mindestens einer Verbindung der Formel (I) nach einem der Ansprüche 1 bis 4 gebildet wird.

**15.** Dispersion nach Anspruch 14, **dadurch gekennzeichnet, dass** die Lipidmembran eine wässerige Phase einkapselt.

**16.** Dispersion nach einem der Ansprüche 14 oder 15, **dadurch gekennzeichnet, dass** die Lipidphase, die die Lipidmembran bildet, ferner mindestens ein nichtionisches amphiphiles Lipid und/oder ein ionisches amphiphiles Lipid enthält.

**17.** Dispersion nach einem der Ansprüche 14 bis 16, **dadurch gekennzeichnet, dass** die Lipidphase, die die Lipidmembran bildet, mindestens einen Zusatzstoff enthält, dessen Hauptfunktion darin besteht, die Permeabilität der Vesikel herabzusetzen, die Flockung und die Verschmelzung der Vesikel zu verhindern und den Grad der Verkapselung zu erhöhen.

**18.** Dispersion nach Anspruch 17, **dadurch gekennzeichnet, dass** der Zusatzstoff unter Sterinen, langkettigen Alkoholen und Diolen, langkettigen Aminen und den quartären Ammoniumderivaten dieser Verbindungen ausgewählt ist.

**19.** Wässerige Dispersion von Vesikeln nach Anspruch 18, **dadurch gekennzeichnet, dass** es sich bei dem Zusatzstoff um Cholesterin handelt.

**20.** Kosmetische oder pharmazeutische Zusammensetzung, **dadurch gekennzeichnet, dass** sie in einem kosmetisch oder pharmazeutisch akzeptablen Medium mindestens eine wässerige Dispersion von Vesikeln nach einem der Ansprüche 14 bis 19 enthält.

**21.** Verwendung einer Verbindung der Formel (I) nach einem der Ansprüche 1 bis 4 als amphiphiles Lipid zur Bildung von Lipidvesikeln.

**Claims**

**1.** Histidine derivative of general formula (I) below:

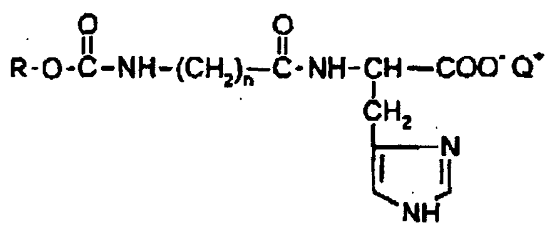

(I)

in which:

R denotes a linear or branched, saturated or unsaturated alkyl radical containing from 6 to 22 carbon atoms,
n is an integer ranging from 1 to 16,
$Q^+$ represents $H^+$ or an organic or inorganic cation,

and the addition salts with an acid, the said organic cation being chosen from ammoniums containing a residue chosen from basic amino acids and amino alcohols, the said inorganic cation being chosen from the group formed by alkali metal salts, alkaline-earth metal salts and the $NH_4^+$ ion and the said addition salts with an acid being chosen from the hydrochiorides, hydrobromides, sulphates, tartrates and acetates.

**2.** Histidine derivative according to Claim 1, in which R denotes a linear or branched, saturated alkyl radical containing

from 8 to 18 carbon atoms.

**3.** Histidine derivative according to either of the preceding claims, in which n is an integer ranging from 1 to 11.

**4.** Histidine derivative according to any one of the preceding claims, chosen from:

- N-octyloxycarbonyl-β-alanyl-L-histidine,
- N-dodecyloxycarbonyl-β-alanyl-L-histidine,
- N-2-ethylhexyloxycarbonyl-β-alanyl-L-histidine hydrochloride,
- N-hexadecyloxycarbonyl-β-alanyl-L-histidine.

**5.** Process for preparing the compounds of general formula (I) below:

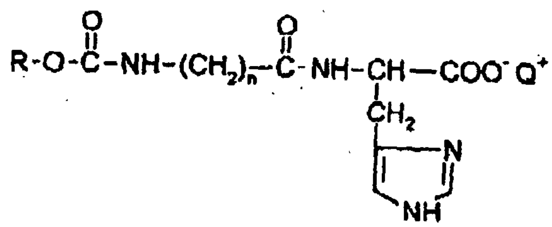

(I)

in which:

R denotes a linear or branched, saturated or unsaturated alkyl radical containing from 6 to 22 carbon atoms,
n is an integer ranging from 1 to 16,
$Q^+$ represents $H^+$ or an organic or inorganic cation,

**characterized in that** it consists in reacting, in an inert solvent, a compound of formula (II)

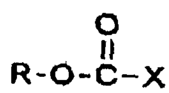

(II)

X representing a halogen atom or a radical derived from an imidazole,

either (A) with carnosine,
or (B), in a first step, with an amino acid of formula:

$$H_2N\text{-}(CH_2)_n\text{-}COOH$$

to form a compound of formula (IV) below:

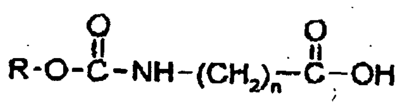

(IV)

and, in a second step, in reacting histidine with the compound of formula (IV) in the presence of a coupling agent.

**6.** Process according to Claim 5, **characterized in that** X is a chlorine atom.

**7.** Composition, **characterized in that** it comprises, in a physiologically acceptable medium, at least one compound of formula (I) as defined in one of Claims 1 to 4.

**8.** Composition according to Claim 7, **characterized in that** it is in the form of a cosmetic or pharmaceutical composition.

**9.** Composition according to either of Claims 7 and 8, **characterized in that** it is in the form of a lotion, a gel, an emulsion, a microemulsion, a milk, a cream, a powder, a paste, a solid stick, a spray or an aerosol mousse.

**10.** Composition according to one of Claims 7 to 9, **characterized in that** the compound of formula (I) is present at a concentration of from 0.01% to 15% by weight relative to the total weight of the composition.

**11.** Use of the compounds of formula (I) according to one of Claims 1 to 4, as free-radical scavengers.

**12.** Use of the compounds of formula (I) according to one of Claims 1 to 4, as free-radical scavengers for deactivating singlet oxygen.

**13.** Use of the compounds of formula (I) according to one of Claims 1 to 4, for the preparation of a cosmetic or pharmaceutical composition intended for treating keratin substances against the effects of ageing.

**14.** Aqueous dispersion of lipid vesicles, **characterized in that** the vesicles contain a lipid membrane formed from at least one compound of formula (I) according to one of Claims 1 to 4.

**15.** Dispersion according to Claim 14, **characterized in that** the lipid membrane encapsulates an aqueous phase.

**16.** Dispersion according to either of Claims 14 and 15, **characterized in that** the lipid phase constituting the lipid membrane also comprises at least one nonionic amphiphilic lipid and/or one ionic amphiphilic lipid.

**17.** Dispersion according to one of Claims 14 to 16, **characterized in that** the lipid phase constituting the lipid membrane comprises at least one additive whose main function is to reduce the permeability of the vesicles, to prevent their flocculation and fusion, and to increase the degree of encapsulation.

**18.** Dispersion according to Claim 17, **characterized in that** the additive is chosen from the group formed by sterols, long-chain alcohols and diols, long-chain amines and the quaternary ammonium derivatives thereof.

**19.** Aqueous dispersion of vesicles according to Claim 18, **characterized in that** the additive is cholesterol.

**20.** Cosmetic or pharmaceutical composition, **characterized in that** it comprises, in a cosmetically or pharmaceutically acceptable medium, at least one aqueous dispersion of vesicles according to one of Claims 14 to 19.

**21.** Use of a compound of formula (I) according to one of Claims 1 to 4, as an amphiphilic lipid to form lipid vesicles.